# EUROPEAN PATENT APPLICATION

(11) **EP 2 712 675 A1**
(43) Date of publication of application: **02.04.2014**
(21) Application number: 12006767.3
(22) Date of filing: 27.09.2012
(51) Int. Cl.: B01J 23/36, B01J 23/62, B01J 35/10, B01J 37/02, B01J 37/06, B01J 37/08, C07C 5/00, C07C 5/32, C07C 5/333

(54) **Alkane dehydrogenation catalyst and process for its preparation**

(71) Applicant: Saudi Basic Industries Corporation, Riyadh 11422 (SA)
(72) Inventor: The designation of the inventor has not yet been filed
(74) Representative: Modiano, Micaela Nadia

(57) **Abstract**

The invention relates to a catalyst composition comprising (i) a porous metal oxide catalyst support, (ii) a noble metal selected from the group consisting of platinum (Pt), palladium (Pd), rhodium (Rh), rhenium (Re), ruthenium (Ru) and iridium (Ir), wherein the amount of noble metal is from 0.1 to 5 wt% based on the porous metal oxide catalyst support and (iii) tin (Sn), wherein the amount of tin is from 0.1 to 5 wt% based on the porous metal oxide catalyst support and (iv) zinc (Zn), wherein the amount of zinc is from 0.1 to 5 wt% based on the porous metal oxide catalyst support and/or (v) an alkaline earth metal, wherein the amount of alkaline earth metal is from 0.1 to 5 wt% based on the porous metal oxide catalyst support, wherein the catalyst composition is obtained or obtainable by a process comprising the steps of (a) depositing the noble metal, Sn, Zn and/or the alkaline earth metal on the porous metal oxide catalyst support to obtain a catalyst precursor and (b) subjecting the catalyst precursor to calcination in an environment comprising oxygen to obtain a catalyst, wherein step (a) comprises the step of (a1) contacting the porous metal oxide catalyst support with a solution comprising a salt of the noble metal and a salt of tin (Sn) and a salt of zinc (Zn) and/or a salt of the alkaline earth metal.

## Description

The invention relates to a catalyst composition suitable for the non-oxidative dehydrogenation of alkanes, to a process for the preparation thereof and to a non-oxidative dehydrogenation process using said catalyst composition and to the use of said catalyst composition in the non-oxidative dehydrogenation of alkanes, preferably of propane.

Alkenes, such as propylene are basic chemicals which are used in industrial processes such as the production of polypropylene, acrylic acid, acrylonitrile, cumene and many others. The demand for alkenes, such as propylene increases annually. Therefore, there is a continuing need to improve the processes for the preparation of alkenes. One such process for the preparation of alkenes, such as propylene is the non-oxidative catalytic dehydrogenation of alkanes, such as propane.

Such a process is for example described in EP0328507A1. EP0328507 discloses a process for the catalytic dehydrogenation of propane, in the presence of hydrogen in a molar ratio of from 0.05 to 0.5 mole of hydrogen per mole of propane over a catalyst consisting of an alumina support containing at least one metal of the platinum group together with a co-catalyst and a promoter, which comprises the step of passing the feed to be dehydrogenated onto a catalyst containing from 0.2 to 1% by weight of platinum, from 0.15 to 1% by weight of tin as co-catalyst and from 0.8 to 2% by weight of potassium as promoter, said catalyst being obtained by submitting the alumina support containing the co-catalyst and calcined at a temperature comprised between 450 and 550°C,
- to a first treatment with a platinum compound, said first treatment being followed by a calcination in air and a reduction in the presence of hydrogen at a temperature comprised between 450 and 550°C;
- then to an intermediate treatment to deposit potassium, said intermediate treatment being followed by a calcination at a temperature comprised between 380 and 550°C,
- and finally to a second treatment with a platinum compound, said second treatment being followed by a calcination at a temperature not exceeding 525°C,
the dehydrogenation being carried out in the presence of said catalyst at a temperature comprised between 530°C and 650°C, a pressure comprised between 0.5 and 3 atm. and a weight hourly space velocity comprised between 1 and 10.

It is the object of the invention to provide an improved process for the non-oxidative dehydrogenation of alkanes.

The object of the invention is achieved by a catalyst composition suitable for the non-oxidative dehydrogenation of alkanes, preferably propane comprising
(i) a porous metal oxide catalyst support,
(ii) a noble metal selected from the group consisting of platinum (Pt), palladium (Pd), rhodium (Rh), rhenium (Re), ruthenium (Ru) and iridium (Ir), wherein the amount of noble metal is from 0.1 to 5 wt% based on the porous metal oxide catalyst support and
(iii) tin (Sn), wherein the amount of tin is from 0.1 to 5 wt% based on the porous metal oxide catalyst support and
(iv) zinc (Zn), wherein the amount of zinc is from 0.1 to 5 wt% based on the porous metal oxide catalyst support and/or
(v) an alkaline earth metal, wherein the amount of alkaline earth metal is from 0.1 to 5 wt% based on the porous metal oxide catalyst support
wherein the catalyst composition is obtained or obtainable by a process comprising the steps of
(a) depositing the noble metal, Sn, Zn and/or the alkaline earth metal on the porous metal oxide catalyst support to obtain a catalyst precursor and
(b) subjecting the catalyst precursor to calcination in an environment comprising oxygen to obtain a catalyst, wherein step (a) comprises the steps of
   (a1) contacting the porous metal oxide catalyst support with a solution comprising a salt of the noble metal and a salt of tin (Sn) and a salt of zinc (Zn) and/or a salt of the alkaline earth metal

The catalyst compositions of the invention can be prepared using an easier process while maintaining their catalytic properties.

In particular, the catalyst composition of the invention is suitable for the non-oxidative dehydrogenation of alkanes, such as propane to alkenes, such as propene. The catalyst composition of the invention may perform this non-oxidative dehydrogenation with a high yield and/or a high selectivity. Furthermore, the catalyst composition of the invention may be more stable for prolonged periods of use.

By using the catalyst composition of the invention in non-oxidative dehydrogenation of alkanes and in particular of propane, one or more of the following additional advantages may also be achieved:
1) the amount of cokes formed on the catalyst composition may be maintained or even reduced
2) the amount of ethylene obtained as a side product (as compared to the total side product) may be increased, thereby increasing the amount of valuable products formed and/or
3) the active surface of the catalyst in the catalyst composition may be increased.

As used herein, the term "catalyst composition" is understood to mean a composition consisting of the catalyst (active phase) and any other suitable components. The catalyst composition of the invention is for example suitable for the non-oxidative dehydrogenation of an alkane and for example particularly suitable for the non-oxidative dehydrogenation of propane.

Examples of porous metal oxide catalyst supports are known to the person skilled in the art and include but are not limited to γ-alumina (γ-Al₂O₃), titania (TiO₂), ceria (CeO₂), zirconia (ZrO₂) and mixtures thereof, that is any mixtures of any one of these porous metal oxide catalyst supports. Preferably, the catalyst composition of the invention comprises γ-alumina (γ-Al₂O₃).

The porous metal oxide catalyst support does not include zeolite supports.

The porous metal oxide catalyst support preferably has a BET surface area of 50-500m²/g, for example a BET surface area of at least 50, for example at least 100, for example at least 150 and/or at most 350, for example at most 250m²/g, for example a BET surface area of 150 to 250m²/g.
As used herein, the BET surface area is measured by the standard BET nitrogen test according to ASTM D-3663-03, ASTM International, October 2003.

In the catalyst composition of the invention, the noble metal is selected from the group consisting of platinum (Pt), palladium (Pd), rhodium (Rh), rhenium (Re), ruthenium (Ru) and iridium (Ir). Preferably, the noble metal is platinum (Pt).

In the catalyst composition of the invention, the noble metal is preferably present in an amount of at least 0.1 wt%, for example at least 0.5wt% based on the porous metal oxide catalyst support and/or at most 5wt%, for example at most 2wt% based on the porous metal oxide catalyst support. For example, the amount of noble metal is in the range of from 1 to 5wt% based on the porous metal oxide catalyst support or in the range of from 0.5 to 2wt% based on the porous metal oxide catalyst support.

In the catalyst composition of the invention, tin (Sn) is preferably present in an amount of at least 0.1wt%, for example at least 0.5wt% based on the porous metal oxide catalyst support and/or at most 5wt%, for example at most 2wt% based on the porous metal oxide catalyst support. For example, the amount of tin (Sn) is in the range of from 1 to 5wt% based on the porous metal oxide catalyst support or in the range of from 0.5 to 2wt% based on the porous metal oxide catalyst support.

In the catalyst composition of the invention, zinc (Zn) is preferably present in an amount of at least 0.1wt%, for example at least 0.5wt% based on the porous metal oxide catalyst support and/or at most 5wt%, for example at most 2wt% based on the porous metal oxide catalyst support. For example, the amount of zinc (Zn) is in the range of from 1 to 5wt% based on the porous metal oxide catalyst support or in the range of from 0.5 to 2wt% based on the porous metal oxide catalyst support.

In the catalyst composition of the invention, the alkaline earth metal is preferably present in an amount of at least 0.1wt%, for example at least 0.5wt% based on the porous metal oxide catalyst support and/or at most 5wt%, for example at most 2wt% based on the porous metal oxide catalyst support. For example, the amount of the alkaline earth metal is in the range of from 1 to 5wt% based on the porous metal oxide catalyst support or in the range of from 0.5 to 2wt% based on the porous metal oxide catalyst support.

Preferably, the alkaline earth metal is selected from the group consisting of magnesium (Mg), calcium (Ca) and strontium (Sr). More preferably, the alkaline earth metal is calcium (Ca).

With 'depositing' is meant herein any technique that can place the noble metal and Sn and Zn and/or the alkaline earth metal on the porous metal oxide catalyst support, such as for example impregnation precipitation, deposition-precipitation, co-precipitation, incipient wetness impregnation or a combination thereof.

The salt solution(s) used in step (a1) to deposit the noble metal and Sn and Zn and/or the alkaline earth metal on the porous metal oxide catalyst support preferably has a pH in the range from 2 to 10, preferably from 4 to 7.5.

The modified slurry may be dried before washing the modified slurry with the solvent. The solvent may be any solvent that is suitable for removal of the anions. For example, water may be used.

Before subjecting the catalyst precursor to calcination in an environment comprising oxygen, the catalyst precursor may (also) be dried.

Drying of the modified slurry and/or of the catalyst precursor may for example be performed by subjecting the modified slurry and/or the catalyst precursor to a temperature of 600-300°C for for example a time period from 0.5 to 6 hours.

In principle, any salt of the noble metal and a salt of tin (Sn) and a salt of zinc (Zn) and/or a salt of the alkaline earth metal, that is soluble in the selected solvent that is used in the solution comprising a salt of the noble metal and a salt of tin (Sn) and a salt of zinc (Zn) and/or a salt of the alkaline earth metal may be used to contact with the porous metal oxide support. For example, suitable salts may be in the form of acetate, oxalate, nitrate, chloride, carbonate, and bicarbonate.

Preferably, one or more of the salts in the solution comprising a salt of the noble metal and a salt of tin (Sn) and a salt of zinc (Zn) and/or a salt of the alkaline earth metal are chloride salts, preferably all salts in said solution are chloride salts.

In case all salts in said solution are chloride salts, the resulting modified slurry may be washed with deionized water until a standard silver nitrate test for the presence of Cl⁻ in the filtrate water is negative.

For example, the salt of the noble metal, for example platinum may be a chloride salt of the noble metal, for example platinum chloride.
For example, the salt of tin may be tin chloride.
For example, the salt of zinc may be zinc chloride. For example, the salt of the alkaline earth metal may be a chloride salt of the alkaline earth metal, for example calcium chloride.

Preferably, step (a) further comprises the step(s) of
(a2) evaporating the liquid in said solution to prepare a modified slurry subsequently after step (a1) and optionally
(a3) washing the modified slurry with a solvent to obtain the catalyst precursor.

Step (b) of the process of the invention, is preferably performed by subjecting the catalyst precursor to calcination in an environment comprising oxygen at a temperature from 100 to 650°, for example a temperature from 400 to 650°C, for example at a time from 1 to 6 hours.

The environment comprising oxygen may for example be achieved using an air stream during the calcination.

In a first special embodiment, the invention relates to catalyst composition comprising
(i) a porous metal oxide catalyst support,
(ii) platinum (Pt), wherein the amount of platinum is from 0.1 to 5 wt% based on the porous metal oxide catalyst support and
(iii) tin (Sn), wherein the amount of tin is from 0.1 to 5 wt% based on the porous metal oxide catalyst support and
(iv) zinc (Zn), wherein the amount of zinc is from 0.1 to 5 wt% based on the porous metal oxide catalyst support and/or
(v) magnesium (Mg), calcium (Ca) or strontium (Sr), wherein the amount of magnesium, calcium or strontium is from 0.1 to 5wt% based on the porous metal oxide catalyst support, wherein the catalyst composition is obtained or obtainable by a process comprising the steps of
   (a) depositing the noble metal, Sn, Zn and/or the alkaline earth metal on the porous metal oxide catalyst support to obtain a catalyst precursor and
   (b) subjecting the catalyst precursor to calcination in an environment comprising oxygen to obtain a catalyst, wherein step (a) comprises the steps of
      (a1) contacting the porous metal oxide catalyst support with a solution comprising a salt of the noble metal and a salt of tin (Sn) and a salt of zinc (Zn) and/or a salt of the alkaline earth metal.

In a second special embodiment, the invention relates to a catalyst composition comprising
(i) a porous metal oxide catalyst support, preferably γ-alumina
(ii) platinum (Pt), wherein the amount of platinum is from 0.1 to 5 wt% based on the porous metal oxide catalyst support
(iii) tin (Sn), wherein the amount of tin is from 0.1 to 5 wt% based on the porous metal oxide catalyst support
(iv) zinc (Zn), wherein the amount of zinc is from 0.1 to 5 wt% based on the porous metal oxide catalyst support and/or
(v) calcium (Ca), wherein the amount of calcium is from 0.1 to 5 wt% based on the porous metal oxide catalyst support
wherein the catalyst composition is obtained or obtainable by a process comprising the steps of
(a) depositing the noble metal, Sn, Zn and/or the alkaline earth metal on the porous metal oxide catalyst support to obtain a catalyst precursor and
(b) subjecting the catalyst precursor to calcination in an environment comprising oxygen to obtain a catalyst, wherein step (a) comprises the steps of
   (a1) contacting the porous metal oxide catalyst support with a solution comprising a salt of the noble metal and a salt of tin (Sn) and a salt of zinc (Zn) and/or a salt of the alkaline earth metal.

In the invention and preferably in these special embodiments of the invention, platinum is preferably the only noble metal present in the catalyst compositions.

Alternatively or also, in the invention, preferably in these special embodiments of the invention, one of magnesium, calcium or strontium is preferably the only alkaline earth metal present in the catalyst composition.

In another aspect, the invention relates to a process for the preparation of a catalyst composition suitable for the non-oxidative dehydrogenation of alkanes, preferably propane comprising
(i) a porous metal oxide catalyst support,
(ii) a noble metal selected from the group consisting of platinum (Pt), palladium (Pd), rhodium (Rh), rhenium (Re), ruthenium (Ru) and iridium (Ir), wherein the amount of noble metal is from 0.1 to 5 wt% based on the porous metal oxide catalyst support and
(iii) tin (Sn), wherein the amount of tin is from 0.1 to 5 wt% based on the porous metal oxide catalyst support and
(iv) zinc (Zn), wherein the amount of zinc is from 0.1 to 5 wt% based on the porous metal oxide catalyst support and/or
(v) an alkaline earth metal, wherein the amount of alkaline earth metal is from 0.1 to 5 wt% based on the porous metal oxide catalyst support
   comprising the steps of
   (a) depositing the noble metal, Sn, Zn and/or the alkaline earth metal on the porous metal oxide catalyst support to obtain a catalyst precursor and
   (b) subjecting the catalyst precursor to calcination in an environment comprising oxygen to obtain a catalyst, wherein step (a) comprises the steps of
      (a1) contacting the porous metal oxide catalyst support with a solution comprising a salt of the noble metal and a salt of tin (Sn) and a salt of zinc (Zn) and/or a salt of the alkaline earth metal

Preferably in said process, step (a) further comprises the steps of
(a2) evaporating the liquid in said solution to prepare a modified slurry subsequently after step (a1) and optionally
(a3) washing the modified slurry with a solvent to obtain the catalyst precursor.

Further details about this process are presented herein.

Until now, catalyst compositions suitable for non-oxidative dehydrogenationof alkanes have been prepared using multiple impregnation steps. It has now been found that it is possible to prepare catalysts compositions non-oxidative dehydrogenation of an alkane by simultaneous impregnation of all active components of the catalyst. Therefore, next to providing new catalyst compositions, the invention also provides a very simple (since it does not require multiple impregnation steps) and effective method for the preparation of these catalyst compositions.

In another aspect, the invention relates to a process for producing an alkene by non-oxidative dehydrogenation of an alkane comprising the step of contacting a feed stream comprising the alkane, preferably propane with the catalyst composition of the invention to form the alkene.

In the framework of the invention, with alkane is meant a hydrocarbon of formula C₂H₂ₙ₊₂. For example, the alkane can have from 2 to 12, preferably from 2 to 4 carbon atoms per molecule. For example, the alkane may be propane, butane, pentane, hexane, heptane, octane, nonane, decane or a mixture thereof. Preferably, the alkane is propane.

Examples of alkenes that may be produced in the process of the invention include but are not limited to propene (also referred to herein as propylene) and ethylene (also referred to herein as ethene) and butene.

The alkane may be used in its pure form, but may also be present in a feedstream of a mixture of alkanes or in a feedstream of alkane (also referred to herein as alkane feedstream) with an inert gas, such as N₂. Preferably, the alkane is present in a feedstream that predominantly comprises one alkane species.

Accordingly, it is preferred that the alkane comprised in the feedstream consists of at least 75 mol % of only one alkane species, more preferably of at least 85 mol % of only one alkane species, even more preferably of at least 90 mol % of only one alkane species, particularly preferably of at least 95 mol % of only one alkane species and most preferably of at least 98 mol % of only one alkane species.

Preferably, the total amount of alkane in the feedstream is at least 98 wt%, preferably at least 99 wt%, for example at least 99.5 wt%, for example at least 99.7 wt%, for example 99.9 wt% based on the total feedstream. Small amounts of olefins (for example from 0.1 to 0.5wt% based on the total feedstream) may be present in the feedstream.

The feedstream may also comprise hydrogen. For example, the molar ratio of hydrogen to alkane in the feedstream may be in the range from about 1:6 to 0:1.

The feedstream may also comprise an inert gas diluent. The inert gas diluent may be chosen from the group of helium, nitrogen, and mixtures thereof, preferably nitrogen. For example, the molar ratio of alkane to inert gas diluent may be in the range from about 1:10 to about 1:1.

As used herein, the term "non-oxidative dehydrogenation" is understood to mean that the dehydrogenation proceeds substantially in the absence of an oxidizing agent, such as oxygen, i.e. the amount of oxidizing agent in a feed stream comprising the alkane is at most 1 vol%, for example at most 0.1 vol% based on the feed stream.

The process of the present invention is performed at conditions suitable for high conversion of an alkane to an alkene. Such conditions are known by the person skilled in the art. Optimal conditions can easily be determined by the person skilled in the art using routine experimentation.

The step of contacting the feed stream comprising the alkane with the catalyst composition of the invention may for example be performed in a reactor at a temperature from 500 to 650°C. Preferably, the step of contacting the feed stream comprising the alkane with the catalyst composition of the invention is performed at a temperature of from 400 to 650, preferably at a temperature from 550 to 650°C, for example at a temperature of at most 575°C, for example at a temperature from 575 to 625°C. A lower temperature has the advantage that the energy required for the non-oxidative dehydrogenation is also lower.

The pressure within the reactor in which the non/oxidative dehydrogenation is performed preferably lies within a range of from 50.7 kilopascals (KPa) to 505kilopascals, more preferably from 40 KPa to 80 KPa. For example, the pressure is 0.01 - 0.3 MPa.

The gas hourly space velocity (GHSV), that is the flow rate at which the feedstream comprising the alkene is fed to the reactor in which the alkane is contacted with the catalyst composition of the invention is for example in the range from 1500 to 6000, for example around 3800h⁻¹.

GHSV is the ratio of the rate at which the feedstream comprising the alkane is fed to the reactor (in volume at standard pressure (101 KPa) per hour divided by the volume of catalyst composition at 101 KPa; and is thus inversely related to contact time.

The weight hourly space velocity (WHSV), that is the ratio of the weight of the alkane which comes in contact with a given weight of catalyst per unit time, is for example in the range from 0.1 to 10 hour⁻¹, for example the weight hourly space velocity is 0.1 to 1 hour⁻¹.

By contact time is meant the period of time during which the alkane feedstream is in contact with the catalyst composition.

Preferably, the step of contacting the feed stream comprising the alkane with the catalyst composition of the invention (the non-oxidative dehydrogenation) is performed at a temperature of from 400 to 650°C, a weight hourly space velocity of 0.1-1 hour⁻¹ and/or a pressure of 0.01 - 0.3 MPa.

The GHSV indicates that there is a certain rate at which the feedstream is fed to the reactor in which the feed stream is contacted with the catalyst composition of the invention. The total length of time in which the feedstream is fed to the reactor is known as the "Time-on-Stream (TOS)." For example the TOS for a catalyst composition according to the invention during which time the catalyst composition maintains its activity in terms of a high conversion and high selectivity for an alkene, for example propylene, ranges from for example 50 to 100 hour.

The step of contacting the alkane with the catalyst composition of the invention may be performed in any suitable reactor, as known to a skilled man, for example in a fixed bed or moving bed reactor.

In another aspect, the invention relates to the use of the catalyst composition of the invention in a non-oxidative dehydrogenation of an alkane, preferably propane.

In another aspect, the invention relates to use of the catalyst composition of the invention in a non-oxidative dehydrogenation of an alkane.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.
It is noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention will now be elucidated by way of the following examples without however being limited thereto.

### EXAMPLES

### Example 1.

### Preparation of 1.0Ca-1.0Zn-1.0Sn-1.0Pt/y-Al₂O₃ catalyst by sequential impregnation

3 grams of γ-Al₂O₃ was dried at 120 °C for two hours. 0.0830g of CaCl₂ was dissolved in 10 ml of deionized (DI) water and the transparent solution was heated to 65 °C, when temperature was stable, pre-heated support was added and the slurry was kept on Rotavapor for 3.5 hours. Then the solution was evaporated under vacuum until only solid slurry was left. This slurry was then dried at 120 °C for two hour in an oven. The powder so obtained was subjected to impregnation with zinc in next step.

0.0647 grams of ZnCl₂ were dissolved in 10 ml of DI water and the transparent solution was heated to 65 °C, when temperature was stable, Ca impregnated gamma alumina was added to the evaporating flask and the slurry was kept on Rotavapor for 3.5 hours. Then the solution was evaporated under vacuum until only solid slurry was left. This slurry was then dried at 120 °C for two hour in an oven. The powder so obtained was subjected to impregnation with tin in next step.

0.0572 grams of SnCl₂.2H₂O were dissolved in 15 ml of ethanol and the transparent solution was heated to 65 °C, when temperature was stable, Ca-Zn impregnated gamma alumina was added to the evaporating flask and the slurry was kept on Rotavapor for 3.5 hours. This slurry was then dried under vacuum to obtain powder. This powdered material was then dried at 120 °C for two hour in an oven. The powder so obtained was subjected to impregnation with Pt in next step.

0.0518g of PtCl₄ were dissolved in 10 ml of DI water and the transparent solution was heated to 65 °C, when temperature was stable, Ca-Zn-Sn impregnated gamma alumina was added to the evaporating flask and the slurry was kept on Rotavapor for 3.5 hours. This slurry was then dried under vacuum to obtain powder. This catalytic material was then washed with DI water to remove any chlorides, which were confirmed by AgNO₃ test. This powdered material was then dried at 120 °C for two hour in an oven. Later on it was calcined at temperature of 600 °C for six hours. The temperature was achieved at ramp rate of 10°C /minute. The catalyst was now ready for testing.

Total preparation time for this catalyst was 30 hours.

The catalyst prepared by this recipe was tested according to method presented in Example 7. The results are presented in Table 1.

### Example 2

### Preparation of 1.0Ca-1.0Sn- 1.0Pt/γ-Al₂O₃ catalyst by sequential impregnation

3 grams of γ-Al₂O₃ was dried at 120 °C for two hours. 0.0830g of CaCl₂ was dissolved in 10 ml of DI water and the transparent solution was heated to 65 °C, when temperature was stable, pre-heated support was added and the slurry was kept on Rotavapor for 3.5 hours. Then the solution was evaporated under vacuum until only solid slurry was left. This slurry was then dried at 120 °C for two hour in an oven. The powder so obtained was subjected to impregnation with Tin in the next step.

0.0572 grams of SnCl₂.2H₂O were dissolved in 15 ml of ethanol and the transparent solution was heated to 65 °C, when temperature was stable, Ca impregnated gamma alumina was added to the evaporating flask and the slurry was kept on Rotavapor for 3.5 hours. This slurry was then dried under vacuum to obtain powder. This powdered material was then dried at 120 °C for two hour in an oven. The powder so obtained was subjected to impregnation with Platinum in the next step.

0.0518g of PtCl₄ were dissolve in 10 ml of DI water and the transparent solution was heated to 65 °C, when temperature was stable, Ca-Sn impregnated gamma alumina was added to the evaporating flask and the slurry was kept on Rotavapor for 3.5 hours. This slurry was then dried under vacuum to obtain powder. This catalytic material was then washed with DI water to remove any chlorides, which were confirmed by AgNO₃ test. This powdered material was then dried at 120 °C for two hour in an oven. Later on it was calcined at temperature of 600 °C for six hours. The temperature was achieved at ramp rate of 10°C /minute. The catalyst was now ready for testing.

Total preparation time for this catalyst was 24 hours.
The catalyst prepared by this recipe was tested according to method presented in Example 7. The results are presented in Table 1.

### Example 3

### Preparation of 1.0Zn-1.0Sn--1.0Pt/γ-Al₂O₃ catalyst by sequential impregnation

3 grams of γ-Al₂O₃ was dried at 120 °C for two hours.0.0647 grams of ZnCl₂ were dissolved in 10 ml of DI water and the transparent solution was heated to 65 °C, when temperature was stable, pre heated gamma alumina was added to the evaporating flask and the slurry was kept on Rotavapor for 3.5 hours. Then the solution was evaporated under vacuum until only solid slurry was left. This slurry was then dried at 120 °C for two hour in an oven. The powder so obtained was subjected to impregnation with Tin in the next step.

0.0572 grams of SnCl₂.2H₂O were dissolved in 15 ml of ethanol and the transparent solution was heated to 65 °C, when temperature was stable, Zn impregnated gamma alumina was added to the evaporating flask and the slurry was kept on Rotavapor for 3.5 hours. This slurry was then dried under vacuum to obtain powder. This powdered material was then dried at 120 °C for two hour in an oven. The powder so obtained was subjected to impregnation with Pt in the next step.

0.0518g of PtCl₄ were dissolve in 10 ml of DI water and the transparent solution was heated to 65 °C, when temperature was stable, Zn-Sn impregnated gamma alumina was added to the evaporating flask and the slurry was kept on Rotavapor for 3.5 hours. This slurry was then dried under vacuum to obtain powder. This catalytic material was then washed with DI water to remove any chlorides, which were confirmed by AgNO₃ test. This powdered material was then dried at 120 °C for two hour in an oven. Later on it was calcined at temperature of 600 °C for six hours. The temperature was achieved at ramp rate of 10°C /minute. The catalyst was now ready for testing.

Total preparation time for this catalyst was 24 hours.

The catalyst prepared by this recipe was tested according to method presented in Example 7. The results are presented in Table 1.

### Example 4

### Preparation of 1.0Pt-1.0Sn-1.0Ca-1.0Zn/γ-Al₂O₃ catalyst by simultaneous impregnation

3 grams of γ-Al₂O₃ was dried at 120 °C for two hours. 0.0518g of PtCl₄ were dissolved in 10 ml of DI water, 0.0572 g of SnCl₂ in 10 ml of ethanol, 0.0725g of CaCl₂ in 10 ml of DI water and 0.0647 grams of ZnCl₂ in 10 ml of DI water. It was assured that solutions of all salts were transparent and there was no suspension at all.

The temperature of the water bath was set to 65°C. The evaporating flask of Rotavapor was filled up with 145 ml of DI water. When the temperature became stable at 65°C, all the metal salts solutions were added to the flask so that the total solution volume becomes 200 ml. The preheated support was added and solution was kept on rotation at this temperature for 3.5 hours. Then the solution was evaporated under vacuum until only solid slurry was left.
The slurry was then dried for 2 hours at 120°C in an oven. The dried catalyst mass was then washed with DI water to remove chloride ions. AgNO₃ test was used to ensure the complete removal of chlorides. The washed catalyst was again dried for two hours at 120 °C and then was calcined at temperature of 600 °C for six hours. The temperature was achieved at ramp rate of 10°C /minute. The catalyst was now ready for testing.

Total preparation time for this catalyst was 12 hours.
The catalyst prepared by this recipe was tested according to method presented in Example 7. The results are presented in Table 2.

### Example 5

### Preparation of 1.0Pt1.0Sn1.0Ca/γ-Al₂O₃ catalyst by simultaneous impregnation

3 grams of γ-Al₂O₃ was dried at 120 °C for two hours. 0.0518g of PtCl₄ were dissolve in 10 ml of DI water, 0.0572 g of SnCl₂ in 10 ml of ethanol, 0.0830g of CaCl₂ in 10 ml of DI water. It was assured that solutions of all salts were transparent and there was no suspension at all. The temperature of the water bath was set to 65 °C. The evaporating flask of Rotavapor was filled up with 145 ml of DI water. When the temperature became stable at 65 °C, all the metal salts solutions were added to the flask so that the total solution volume becomes 200 ml. The preheated support was added and solution was kept on rotation at this temperature for 3.5 hours. Then the solution was evaporated under vacuum until only solid slurry was left.

The slurry was then dried for 2 hours at 120 in the oven. The dried catalyst mass was then washed with DI water to remove chloride ions. AgNO₃ test was used to ensure the complete removal of chlorides. The washed catalyst was again dried for two hours at 120 °C and then was calcined at temperature of 600 °C for six hours. The temperature was achieved at ramp rate of 10°C /minute. The catalyst was now ready for testing.

Total preparation time for this catalyst was 12 hours. The catalyst prepared by this recipe was tested according to method presented in Example 7. The results are presented in Table 2.

### Example 6

### Preparation of 1.0Pt1.0Sn 1.0Zn/γ-Al₂O₃ catalyst by simultaneous impregnation

3 grams of γ-Al₂O₃ was dried at 120 °C for two hours. 0.0518g of PtCl₄ were dissolve in 10 ml of DI water, 0.0572 g of SnCl₂ in 10 ml of ethanol and 0.0647 grams of ZnCl₂ in 10 ml of DI water. It was assured that solutions of all salts were transparent and there was no suspension at all.

The temperature of the water bath was set to 65 °C. The evaporating flask of Rotavapor was filled up with 145 ml of DI water. When the temperature becomes stable at 65°C, all the metal salts solutions were added to the flask so that the total solution volume becomes 200 ml. The preheated support was added and solution was kept on rotation at this temperature for 3.5 hours. Then the solution was evaporated under vacuum until only solid slurry was left.

The slurry was then dried for 2 hours at 120°C in an oven. The dried catalyst mass was then washed with DI water to remove chloride ions. AgNO₃ test was used to ensure the complete removal of chlorides. The washed catalyst was again dried for two hours at 120 °C and then was calcined at temperature of 600 °C for six hours. The temperature was achieved at ramp rate of 10 °C /minute. The catalyst in now ready for testing.

Total preparation time for this catalyst was 12 hours. The catalyst prepared by this recipe was tested according to method presented in Example 7. The results are presented in Table 2.

### Example 7. Testing of the catalytic activity of the catalysts in the reaction of dehydrogenation of propane

The catalytic activity in the reaction propane dehydrogenation of the catalysts prepared according to the methods described in Examples 1-6 were measured using a quartz flow reactor with i.d. = 6 mm containing 0.25 - 1.0 g catalyst mixed with 1.0 g quartz sand (mesh size 12 - 25). The reaction temperature was 575°C, measured by thermocouple located in the catalyst bed. The reacting gas contained C₃H₈, H₂ and N₂. The compositions of inlet reaction gas containing C₃H₈:H₂:N₂ in volume ratio was C₃H₈:H₂:N₂ = 1.0 : 1.0 : 5.0. The Gas Hourly Space Velocity (GHSV) of the reacting gas was 3800 h⁻¹. The flow rates of the gases at reactor inlet were controlled by mass flow controllers.

Before use the catalyst was reduced by hydrogen in the reactor at the temperature of 575°C for 2 hours.

The inlet and outlet composition of the reactants was analyzed by gas chromatograph SRI 8610C (USA) with PID and HWD detectors. The reaction products were separated on 2 m column filled with alkalinized alumina. The carrier gas was nitrogen.

The data from activity tests of catalysts prepared according to Examples 1-6 is presented in Table 1 for catalysts prepared by sequential impregnation of active catalyst components and in Table 2 for catalysts prepared by simultaneous impregnation of active catalyst components.

**Table 1: Activity results of catalysts prepared by sequential impregnation for various catalysts at 575 C, GHSV 3800 h⁻¹ and gas feed ratio C₃H₈:H₂:N₂ = 1:1:5**

| Catalyst | Preparation time (h) | After 1 hour reaction time | | | After 2 hours reaction time | | | Amount of coke mg.g.cat^{- 1}h⁻¹ |
|---|---|---|---|---|---|---|---|---|
| | | C₃H₈ Conversion % | C₃H₆ Selectivity % | C₃H₆ Yield % | C₃H₈ Conversion % | C₃H₆ Selectivity % | C₃H₆ Yield % | |
| PtSnCaZn /γ-Al₂O₃ | 30.0 | 33.8 | 95.8 | 23.8 | 31.1 | 96.2 | 22.1 | 35.2 |
| PtSnCa/ γ-Al₂O₃ | 24.0 | 41.9 | 96.2 | 18.5 | 41.1 | 97.0 | 17.0 | 15.8 |
| PtSnZn/ γ-Al₂O₃ | 24.0 | 45.0 | 96.5 | 21.6 | 45.6 | 96.9 | 22.2 | 11.9 |

**Table 2: Activity results of catalysts prepared by simultaneous impregnation for various catalysts at 575 C, GHSV 3800 h⁻¹ and gas feed ratio C₃H₈:H₂:N₂ = 1:1:5.**

| Catalyst | Preparation time (h) | After 1 hour reaction time | | | After 2 hours reaction time | | | Amount of coke mg.g.cat^{-1 1}h⁻¹ |
|---|---|---|---|---|---|---|---|---|
| | | C₃H₈ Conversion % | C₃H₆ Selectivity % | C₃H₆ Yield % | C₃H₈ Conversion % | C₃H₆ Selectivity % | C₃H₆ Yield % | |
| PtSnCaZn /γ-Al₂O₃ | 12.0 | 44.0 | 94.3 | 33.7 | 42.8 | 95.4 | 32.7 | 8.9 |
| PtSnCa/ γ-Al₂O₃ | 12.0 | 45.0 | 95.0 | 32.5 | 43.8 | 95.2 | 32.1 | 7.1 |
| PtSnZn/ γ-Al₂O₃ | 12.0 | 45.0 | 95.0 | 32.5 | 43.8 | 95.2 | 32.1 | 12.6 |

As can be seen from the results presented in Tables 1 and 2 above, catalysts of the invention can be prepared in less time. Furthermore, they show an equal or improved catalytic activity (for propane dehydrogenation), as can be seen from a maintained or improved conversion, a maintained or improved selectivity or a maintained or improved yield. Furthermore, the catalysts of the invention may also lead to the formation of less cokes.

## Claims

1. Catalyst composition comprising
(i) a porous metal oxide catalyst support,
(ii) a noble metal selected from the group consisting of platinum (Pt), palladium (Pd), rhodium (Rh), rhenium (Re), ruthenium (Ru) and iridium (Ir), wherein the amount of noble metal is from 0.1 to 5 wt% based on the porous metal oxide catalyst support and
(iii) tin (Sn), wherein the amount of tin is from 0.1 to 5 wt% based on the porous metal oxide catalyst support and
(iv) zinc (Zn), wherein the amount of zinc is from 0.1 to 5 wt% based on the porous metal oxide catalyst support and/or
(v) an alkaline earth metal, wherein the amount of alkaline earth metal is from 0.1 to 5 wt% based on the porous metal oxide catalyst support,
wherein the catalyst composition is obtained or obtainable by a process comprising the steps of
(a) depositing the noble metal, Sn, Zn and/or the alkaline earth metal on the porous metal oxide catalyst support to obtain a catalyst precursor and
(b) subjecting the catalyst precursor to calcination in an environment comprising oxygen to obtain a catalyst, wherein step (a) comprises the steps of
(a1) contacting the porous metal oxide catalyst support with a solution comprising a salt of the noble metal and a salt of tin (Sn) and a salt of zinc (Zn) and/or a salt of the alkaline earth metal.

2. Catalyst composition according to claim 1, wherein step (a) further comprises the step(s) of
(a2) evaporating the liquid in said solution to prepare a modified slurry subsequently after step (a1) and optionally
(a3) washing the modified slurry with a solvent to obtain the catalyst precursor.

3. Catalyst composition according to claim 1 or claim 2, wherein all salts in the solution of step (a1) are chloride salts.

4. Catalyst composition according to any one of claims 1-3, wherein the porous metal oxide catalyst support is selected from the group consisting of γ-alumina (γ-Al₂O₃), titania (TiO₂), ceria (CeO₂), zirconia (ZrO₂) and any mixtures thereof, preferably γ-alumina (γ-Al₂O₃).

5. Catalyst composition according to any one of claims 1-4, wherein the noble metal is platinum (Pt).

6. Catalyst composition according to any one of claims 1-5, wherein the alkaline earth metal is selected from the group consisting of magnesium (Mg), calcium (Ca) and strontium (Sr), preferably calcium (Ca).

7. Catalyst composition according to any one of claims 1-6, wherein the porous metal oxide catalyst support has a BET surface area of 50- 500m²/g.

8. Process for the preparation of a catalyst composition comprising
(i) a porous metal oxide catalyst support,
(ii) a noble metal selected from the group consisting of platinum (Pt), palladium (Pd), rhodium (Rh), rhenium (Re), ruthenium (Ru) and iridium (Ir), wherein the amount of noble metal is from 0.1 to 5 wt% based on the porous metal oxide catalyst support and
(iii) tin (Sn), wherein the amount of tin is from 0.1 to 5 wt% based on the porous metal oxide catalyst support and
(iv) zinc (Zn), wherein the amount of zinc is from 0.1 to 5 wt% based on the porous metal oxide catalyst support and/or
(v) an alkaline earth metal, wherein the amount of alkaline earth metal is from 0.1 to 5 wt% based on the porous metal oxide catalyst support
comprising the steps of
(a) depositing the noble metal, Sn, Zn and/or the alkaline earth metal on the porous metal oxide catalyst support to obtain a catalyst precursor and
(b) subjecting the catalyst precursor to calcination in an environment comprising oxygen to obtain a catalyst, wherein step (a) comprises the steps of
(a1) contacting the porous metal oxide catalyst support with a solution comprising a salt of the noble metal and a salt of tin (Sn) and a salt of zinc (Zn) and/or a salt of the alkaline earth metal

9. Process according to claim 8, wherein step (a) further comprises the steps of
(a2) evaporating the liquid in said solution to prepare a modified slurry subsequently after step (a1) and optionally
(a3) washing the modified slurry with a solvent to obtain the catalyst precursor.

10. Process for producing an alkene by non-oxidative dehydrogenation of an alkane comprising the step of contacting a feed stream comprising the alkane with the catalyst composition of any one of claims 1-7 to form the alkene.

11. Process according to claim 10, wherein the alkane is propane.

12. Process according to claim 10 or claim 11, wherein the non-oxidative dehydrogenation is performed at a temperature of from 400 to 650°C, a weight hourly space velocity of 0.1-1 hour⁻¹ and/or a pressure of 0.01 - 0.3MPa.

13. Use of the catalyst composition of any one of claims 1-7 in a non-oxidative dehydrogenation of an alkane.

14. Use according to claim 13, wherein the alkane is propane.
